# EUROPEAN PATENT APPLICATION

(11) **EP 1 380 278 A1**
(43) Date of publication of application: **14.01.2004**
(21) Application number: 02713266.1
(22) Date of filing: 29.03.2002
(51) Int. Cl.: A61K 7/00, A61K 7/42, A61K 7/48, A61K 9/107, A61K 47/34, A61K 47/16, A61K 47/28, A61K 47/24, A61K 47/10, A61P 17/16

(54) **COSMETIC EMULSION PREPARATION AND AGENT FOR EXTERNAL USE**

(30) Priority: 29.03.2001 JP 2001097440; 29.03.2001 JP 2001097441
(71) Applicant: The Nisshin OilliO, Ltd., Tokyo 104-8285 (JP); Taiyo Kagaku Co., Ltd., Yokkaichi-shi Mie-ken 510-0825 (JP)
(72) Inventor: OOYAMA, Keiichi c/o The Nisshin Oil Mills, Ltd., Yokohama-shi, Kanagawa 235-0023 (JP); FUJINO, Jin c/o The Nisshin Oil Mills, Ltd., Yokohama-shi, Kanagawa 235-0023 (JP); UCHIDA, Kazuhito c/o Taiyo Kagaku Co., Ltd., Yokkaichi-shi, Mie 510-0825 (JP)
(74) Representative: Vuillermoz, Bruno
(86) International application number: PCT/JP2002/003203
(87) International publication number: WO 2002/078650

(57) **Abstract**

An emulsion which has an average particle diameter of 100 nm or smaller and comprises: (D) a polyglycerol/fatty acid ester which has an HLB of 13 or higher and in which the polyglycerol is one in which the average degree of polymerization is 8 to 12, the total content of the pentamer and hexamer is 50% by weight or higher, and the total content of the tetramer and lower polymers is less than 20% by weight, and the fatty acid residue is C₁₄₋₂₂ hydrocarbon group; (E) a polyglycerol/fatty acid ester which has an HLB of 5 to 11 and in which the polyglycerol is one in which the average degree of polymerization is 2 to 12, the total content of the tetramer and lower polymers is 20% by weight or higher, each of (2/3)n components being contained in an amount of at least 5% by weight or more, where n (n is 2 or more) is taken to be an average degree of polymerization (when (2/3)n is not a natural number, (2/3)n is considered to be a lowest natural number greater than (2/3)n), and the fatty acid residue is a C₁₄₋₂₂ hydrocarbon group; (F) a lecithin; and (G) an oily ingredient.

## Description

### Technical Field

The present invention relates to a cosmetic emulsion preparation and agent for external use, having for its emulsification base a polyglycerol fatty acid ester, which is used in the fields of cosmetics; quasi-drugs, drugs, household articles, and so forth, and more particularly, relates to a cosmetic emulsion preparation and agent for external use having a stable emulsification over a wide temperature range, no large decrease in hardness at high temperatures above 40°C, no increase in hardness with the passage of time even at 0°C, a fine texture in appearance, and smoothly vanishing without leaving behind any white residue on the skin when applied. In addition, the present invention relates to a transparent cosmetic emulsion preparation and agent for external use, having lecithin in addition to a polyglycerol fatty acid ester for its emulsification base, and more particularly, relates to a transparent cosmetic emulsion preparation and agent for external use having an average particle diameter of 100 nm or less, a clear appearance; being stable without the occurrence of decreased transparency, clouding, or ingredient separation with the passage of time; and exhibiting hardly any lowering of pH as is frequently observed in aqueous compositions combining the use of polyglycerol fatty acid ester and lecithin.

### Background Art

Polyoxyethylene-based surfactants are typically used as emulsifiers or solubilizers in cosmetic emulsion preparations and solubilized cosmetic preparations in which oily ingredients are finely dispersed, examples of which include cosmetics and agents for external use such as ointments, creams, moisturizer, toner, essence, lotions, and eau de cologne. Reasons for the wide use of polyoxyethylene-based surfactants include their ease of use as emulsifiers or solubilizers, and their structural characteristic of allowing chain length of hydrophilic groups in molecules to be controlled as desired, thereby resulting in products being available on the market having various HLB values. In addition, since the HLB value can be adjusted by a single surfactant or a combination thereof, fine emulsions can be formed easily, thereby facilitating the introduction of phase inversion emulsification. In this method, an emulsion is usually formed by dissolving or dispersing surfactant in an oily phase followed by the addition of aqueous phase to invert the emulsion from a W/O emulsion to an O/W emulsion. In comparison with the dispersion emulsification method in which an aqueous phase is added to an oily phase, since a satisfactory emulsion having a small particle diameter can be obtained, this method is commonly used for production of cosmetics and agents for external use in particular. In addition, the balance between hydrophilic properties and lipophilic properties has been reported to involve lamellar liquid crystal formed during phase inversion ("Journal of Oleo Science", Vol. 30, No. 1,1981).

For example, if only a very small amount of oily ingredient is contained in a cosmetic or agent for external use, it can be solubilized by dissolved into micelles using a surfactant having a high HLB value. The advantages of such cosmetic products and agents for external use include the imparting of a refreshing feeling that is not oily even though they contain an oily ingredient, stable blending of the oily ingredient without having to increase the viscosity of the system and resulting in an attractive appearance. These advantages result in high product value.

However, there are concerns over polyoxyethylene-based surfactants in terms of safety. Polyglycerol fatty acid esters are used as alternative surfactants due to their high level of safety. Polyglycerol, which is a constituent component of many of the polyglycerol fatty acid esters typically on the market, has an average degree of polymerization as determined from the hydroxyl value of 2-10, and HLB can be adjusted to a certain degree by the composition and amounts of esterified fatty acids. This polyglycerol is typically obtained by a dehydration condensation reaction, is inexpensive and is commonly used in food applications.

In addition, polyglycerol fatty acid ester has limitations when considering its use as a hydrophilic surfactant for cosmetic applications. Although the average degree of polymerization of polyglycerol obtained by the aforementioned dehydration condensation reaction is 2-10, the reason for the absence of products on the market having a higher average degree of polymerization is that an insoluble rubber-like substance forms during the reaction when the degree of polymerization becomes high, thereby resulting in a loss of the inherent hydrophilic properties of polyglycerol. This is due to an increase in cyclic and steric bonding when the degree of polymerization increases beyond a certain level. One characteristic of polyglycerol obtained by a dehydration condensation reaction is the wide distribution of the degree of polymerization of the components. A large number of components having a low degree of polymerization can be determined to be present when looking at the distribution of polymerization by gas chromatography or liquid chromatography. Fatty acid esters of polyglycerol have hydrophilic properties that are less hydrophilic than that which would be expected from the average degree of polymerization of polyglycerol.

For example, even in the case of mono fatty acid esters of polyglycerol having an average degree of polymerization of 10 typically available on the market, in the case of stearic acid, the HLB value as determined by an emulsification experiment is not that high at about 11-12. Although the HLB value naturally becomes higher as the chain length of the fatty acid becomes shorter (for example, an HLB value of about 15 in the case of lauric acid), since the emulsification capacity of a surfactant is generally known to be higher for higher fatty acids than medium fatty acids, these are not suitable for use as emulsifiers. In the case of using as a cosmetic or agent for external use in particular, emulsified oil having various polarities and molecular weights are present, and in the case of hydrocarbon oils and ester oils, the polarities and molecular weights of the oily agents are not uniform. In addition, HLB values determined in oily agents having high polarity, such as ester oils and alcohols, are not uniform. In order to produce a fine emulsion, it is necessary to adjust the HLB value to a suitable value by mixing surfactant having a high HLB value and surfactant having a low HLB value.

Since fatty acid esters of polyglycerol conventionally available on the market, which are obtained from a dehydration condensation reaction and have a wide distribution of average degree of polymerization and a high average degree of polymerization, do not have very high HLB values, they are not emulsifiers suitable for ensuring a balance between hydrophilic properties and lipophilic properties, thereby making it difficult to obtain a satisfactory emulsion in the case of using such esters.

In addition, linear, saturated higher alcohols and alkyl glyceryl ethers are commonly used when adjusting the hardness of cosmetic emulsion preparations and agents for external use. Inversion emulsification is effective for adjusting hardness, and liquid crystal is formed at an oil-water interface by using a higher alcohol or other emulsification assistant with a surfactant. This method is preferable for generating the characteristic hardness of creams, moisturizer, and so forth. Emulsification methods such as D-phase emulsification and liquid crystal emulsification have been developed as easy methods for producing emulsions. Although these methods are convenient since they require hardly any adjustment of HLB, since it is difficult to effectively orient higher alcohols at the oil-water interface, adjustment of hardness is difficult and there are many cases in which the desired hardness cannot be produced. Although hardness increases if the blended amount of higher alcohol is increased, the fineness in terms of texture in appearance is worsened, which results in residual white residue on the skin when applied and the shortcoming of the so-called lack of vanishing, thereby resulting in the problem of a product with low product value. The term "vanishing" used here refers to the ability to immediately penetrate into the skin without any white residue remaining on the skin surface.

There are several methods for obtaining highly hydrophilic polyglycerol fatty acid esters. For example, polyglycerol fatty acid esters obtained by a ring-opening polymerization reaction or nucleophilic substitution reaction using glycidol, epichlorohydrin, monochlorohydrin, dichlorohydrin, or so forth as a starting substance have relatively highly hydrophilic as compared with polyglycerol fatty acid esters having an equal average degree of polymerization obtained by a dehydration condensation reaction. The difficulty in cyclization due to the reaction characteristics is a factor behind the high degree of hydrophilic properties of the former. Polyglycerol having few lowly polymerized components and a narrow distribution of the degree of polymerization are obtained by a synthesis method in which the reaction process is controlled (Japanese Unexamined Patent Application, First Publication No. 7-100355). In addition, examples of highly pure fatty acid esters of polyglycerol are disclosed in Japanese Unexamined Patent Application, First Publication No. 62-266135. In addition, a technique for removing components having a low degree of polymerization from polyglycerol by column fractionation has already been developed and industrialized, and such fatty acid esters have been developed (Japanese Unexamined Patent Application, First Publication No. 8-143513).

On the other hand, in recent years a growing number of cosmetic agents have come to use lecithin as a natural surfactant having a high degree of safety. Lecithin not only acts as a surfactant, but is also useful as an ingredient of cosmetics for moisturizing which easily retain moisture due to the formation of lamellar liquid crystal by interacting with water.

When polyglycerol fatty acid ester and lecithin are used in combination in a cosmetic product or agent for external use, the emulsifying capacity is increased more than in the case of using each alone. In addition, polyglycerol fatty acid ester is also used as a dispersant for dispersing lecithin in water.

The effect of the distribution of the degree of polymerization of polyglycerol on the emulsification performance of polyglycerol fatty acid ester has been reported from the aspect of phase behavior (The 37th Oleo Science Panel Discussion, Abstracts of Presentations, p. 109, 1998). According to that report, surfactant density at an oil-water interface is related to the distribution of the degree of polymerization of polyglycerol, with a broad distribution of the degree of polymerization suggesting high surfactant density and high emulsification performance. The case of a narrow distribution of the degree of polymerization or the use of a single polyglycerol fatty acid ester as an emulsifier offers the advantage of allowing the obtaining of a highly hydrophilic polyglycerol fatty acid ester. However, this is inadequate for obtaining a stable cosmetic agent in which oily ingredients are finely emulsified and dispersed due to the relationship between interface density and emulsification performance. In addition, in the aforementioned technology, a composition, in which a higher alcohol or alkyl glyceryl ether is blended in order to adjust the hardness of the cosmetic agent or agent for external use, has the problem resulting in a emulsion that has difficulty in vanishing and allows white residue to remain when applied to the skin.

When the blended amount of emulsifier is increased to avoid this problem, hardness decreases or the presence of the emulsifier can be felt strongly, thereby making this undesirable. In addition, although the polyglycerol ester of a branched fatty acid disclosed in Japanese Unexamined Patent Application, First Publication No. 58-185537 has a lower melting point than polyglycerol fatty acid ester of a saturated, linear higher fatty acid, and exhibits smaller changes in viscosity caused by changes in temperature, the resulting product is soft. If the blended amount of higher alcohol is then increased to enhance hardness, the problem previously described ends up occurring.

On the other hand, a new problem has recently surfaced during the course of the distribution of cosmetic agents and agents for external use as commercial products. The previous guaranteed temperature for high-temperature stability during the distribution of commercial products of cosmetic agents and agents for external use was 40°C. In general, the temperature conditions for shelf life tests used for cosmetic agents and agents for external use is 40°C, and this temperature is also used in accelerated testing. However, due to the recent environmental problems of warming caused by destruction of the ozone layer and the urban heat island phenomenon, average air temperatures have risen from those recorded in the past, and the rise in the air temperature during the day in cities and their suburbs have been particularly remarkable, frequently rising to 35°C or higher. Consequently, the temperature in non-air-conditioned rooms, bags during transport, and so forth frequently exceeds 40°C. As a result, the hardness of cosmetic agents products and agents for external use decreases significantly, frequently resulting in problems in which they flow out rapidly when taken out of their containers, cause leakage from their containers or eventually resulting in separation of the oily layer with the passage of time.

Consequently, the temperature stability of cosmetics and agents for external use is such that it has become necessary to achieve hardness stability and storage stability at temperatures higher than 40°C. This problem cannot be resolved with conventional polyoxyethylene-based surfactants or technologies like those disclosed in Japanese Unexamined Patent Application, First Publication No. 58-185537, and the only alternative is to accommodate the problem by increasing the amounts of higher alcohol and wax ingredients or increasing the amount of thickener. However, such cosmetics and agents for external use having an extremely poor texture and appearance. In addition, when blended in this manner, hardness stability at low temperatures may become poor with the passage of time and the higher alcohol and wax ingredients easily aggregate with the passage of time, resulting in a significant increase in hardness that may ultimately result in elimination of water. This tendency is particularly conspicuous when the ambient temperature of the cosmetic agent or agent for external use falls to 0°C.

Thus, a first object of the present invention is to provide a cosmetic emulsion preparation and agent for external use comprising an emulsified composition having polyglycerol fatty acid ester as its emulsification base, wherein even in the case of using a polyglycerol fatty acid ester having a high HLB value so as to facilitate use in phase inversion emulsification, there is no remarkable decrease in hardness at temperatures higher than 40°C, it remains stable with the passage of time when stored, it is stable with the passage of time and there is no elimination of water even at temperatures of 0°C without increasing in hardness, and it has a satisfactorily fine texture in appearance and vanishes smoothly without leaving any white residue on the skin.

In addition, as was previously described, it is empirically known that the use of polyglycerol fatty acid ester and lecithin in combination results in improved emulsification performance than using each alone, and cosmetic agents and agents for external use that contain both of these as emulsifiers have been studied and marketed. However, these products have the serious problem of pH decreasing with the passage of time. This is thought to occur due to the lecithin typically used in marketed products being of natural origin, and residual trace ingredients of the lecithin promoting a hydrolysis reaction that causes the release of free fatty acid contained in the lecithin. These trace ingredients not only promote hydrolysis of the lecithin, but also of the polyglycerol fatty acid ester, also resulting in the release of fatty acid. Not only does this decrease in pH cause a problem in terms of irritation of the skin during use, but it also causes a remarkable decrease in value as a product having a transparent appearance since fatty acid is released and forms an insoluble precipitate. Moreover, as a result of this decrease in pH, the pH eventually reaches the isoelectric point of lecithin and so forth, causing the lecithin to become insoluble and ultimately leading to breakdown of the emulsion, cloudiness, and separation.

Although studies have been conducted to prevent these problems such as the addition of ingredients having chelating effects (Japanese Unexamined Patent Application, First Publication No. 2-169512), since the amount blended is limited since lecithin salts out easily in the presence of salt, and ingredients having strong chelating effects cause considerable irritation of mucous membranes, there are numerous restrictions on their blending.

Thus, a second object of the present invention is to provide a transparent cosmetic emulsion preparation and agent for external use comprising a transparent and fine cosmetic emulsion preparation having polyglycerol fatty acid ester and lecithin as its emulsification base, which has satisfactory emulsification stability for which there is no decrease in transparency or separation with the passage of time, and has particularly satisfactory pH stability.

Furthermore, the transparent cosmetic referred to here indicates that having transmittance of 50% T or more, and preferably 70% T or more, using purified water as a control and measuring with a spectrophotometer when the measurement wavelength is 750 nm and the measurement light path is 10 mm.

### DISCLOSURE OF INVENTION

As a result of conducting extensive research to solve the aforementioned problems, the inventors of the present invention found that the above problems can be solved with a cosmetic emulsion preparation and agent for external use containing as essential ingredients hydrophilic and lipophilic polyglycerol fatty acid ester, for which HLB, the content of a specific degree of polymerization, fatty acid residues and so forth are specified, and a specific emulsification assistant, thereby leading to completion of the present invention.

Namely, in a first aspect of the present invention, a cosmetic emulsion preparation and agent for external use is provided comprising as essential ingredients: (A) polyglycerol fatty acid ester as hydrophilic surfactant in which the HLB value is 13 or more, the average degree of polymerization of the polyglycerol is 8 to 12, the total content of pentamer and hexamer is 50 % by weight or more, the total content of tetramer and lower polymers is less than 20 % by weight, and the fatty acid residue is a linear alkyl group having 14 to 22 carbon atoms; (B) polyglycerol fatty acid ester as lipophilic surfactant in which the HLB value is 5 to 11, the average degree of polymerization of the polyglycerol is 2 to 12, the total content of tetramer or lower polymer is 20 % by weight or more, each of (2/3)n components being contained in an amount of at least 5% by weight or more, where n (n is 2 or more) is taken to be an average degree of polymerization (when (2/3)n is not a natural number, (2/3)n is considered to be a lowest natural number greater than (2/3)n), and the fatty acid residue is a linear alkyl group having 14 to 22 carbon atoms; and (C) a saturated linear alcohol having 16 to 22 carbon atoms and/or a linear monoalkyl glyceryl ether having 16 to 22 carbon atoms.

Moreover, in a second aspect of the present invention, a transparent cosmetic emulsion preparation and agent for external use, in which the average particle diameter of the emulsion is 100 nm or less, are provided comprising as essential ingredients: (D) polyglycerol fatty acid ester as hydrophilic surfactant in which the HLB value is 13 or more, the average degree of polymerization of the polyglycerol is 8 to 12, the total content of pentamer and hexamer is 50 % by weight or more, the total content of tetramer and lower polymers is less than 20 % by weight, and the fatty acid residue is a linear alkyl group having 14 to 22 carbon atoms; (E) polyglycerol fatty acid ester as lipophilic surfactant in which the HLB value is 5 to 11, the average degree of polymerization of the polyglycerol is 2 to 12, the total content of tetramer or lower polymer is 20 % by weight or more, each of (2/3)n components being contained in an amount of at least 5% by weight or more, where n (n is 2 or more) is taken to be an average degree of polymerization (when (2/3)n is not a natural number, (2/3)n is considered to be a lowest natural number greater than (2/3)n), and the fatty acid residue is a hydrocarbon group having 14 to 22 carbon atoms; (F) lecithin; and (G) an oily ingredient.

Furthermore, the average degree of polymerization referred to here indicates the average degree of polymerization as determined from the hydroxyl value that is commonly used for polyglycerol available on the market. The constituent components of the polyglycerol indicate those that were analyzed by analytical methods such as gas chromatography and liquid chromatography. In addition, the polyglycerol referred to here indicates that which includes mixtures of glycerol polymers having different degrees of polymerization and cases in which unreacted free glycerol remains.

The average degree of polymerization of the polyglycerol in the present invention was determined from the hydroxyl value as defined in Standard Methods for the Analysis of Fats, Oils and Related Materials (edited by Japan Oil Chemists' Society). In addition, in measuring the component composition of each degree of polymerization, it is appropriate to determine the degree of polymerization in terms of the polyglycerol derivative by separating and quantifying by gas chromatography (GC). Analysis by GC can be easily carried out by, for example, analyzing while raising the temperature from 100°C to 250°C at the rate of 10°C/minute using a fused silica capillary tube chemically bonded with a low polarity liquid phase such as methyl silicon. In addition, identification of the degree of polymerization of the peaks on the chromatograms can be easily carried out by, for example, connecting the gas chromatograph to a double-focusing mass spectrograph and measuring by ionizing by chemical ionization or other method, followed by determining the molecular weights of the peaks on the chromatogram from the molecular weight of the parent ion, and finally determining the degree of polymerization of glycerol according to the chemical formula. However, identification is not limited to this method. Furthermore, when calculating the average degree of polymerization of polyglycerol from the hydroxyl value, calculations are made on the hypothesis that polyglycerol having an average degree of polymerization n has (n+2) hydroxyl groups. Since the presence of cyclical polyglycerol and the presence of hydroxyl groups that have difficulty in reaction during analysis are not taken into consideration, the average degree of polymerization is frequently higher than the results of component analysis by gas chromatography or liquid chromatography.

Measurement of the HLB value of polyglycerol fatty acid ester should use a method in which the HLB value is actually measured by performing an emulsification experiment. Namely, an emulsion of liquid paraffin is produced by combining hydrophilic and lipophilic surfactants for which the HLB values are known, followed by calculating the required HLB of the liquid paraffin according to the combination that produces the most favorable oil-in-water emulsion. In this method, the HLB value of polyglycerol fatty acid ester is determined from the ratio at which the most favorable oil-in-water emulsion is produced by emulsifying liquid paraffin with an unknown polyglycerol fatty acid ester and known surfactant, and the aforementioned required HLB. In the present invention, although HLB values were determined relatively using as known surfactants commercially available decaglycerol monolaurate ester (HBL value = 15.0) and diglycerol monoisostearate (HLB value = 5.4), the known surfactants are not limited to these.

Furthermore, although the HLB value of polyglycerol fatty acid ester of the present invention can also be determined directly from the empirical formula of Griffin, a discrepancy occurs as compared with methods for determining HLB values by emulsification experiments (refer to "Polyglycerol Esters", p. 107, edited by Sakamoto Yakuhin Kogyo Co., Ltd., ).

### BRIEF DESCRIPTION OF THE DRAWING

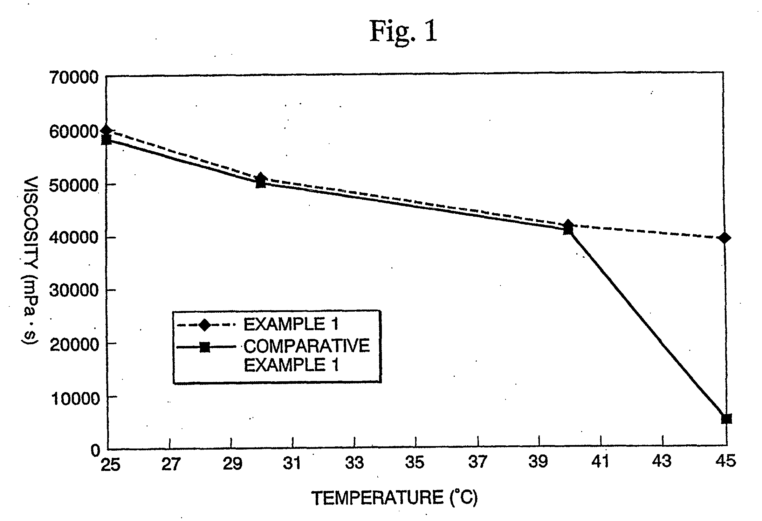

Fig. 1 is a graph showing the viscosity of a cream versus temperature.

### BEST MODE FOR CARRYING OUT THE INVENTION

The following provides an explanation of the constituent components of the present invention. The emulsifier of the present invention requires a combination of polyglycerol fatty acid ester having an HLB value of 13 or more, and polyglycerol fatty acid ester having an HLB value of 5 to 11, and is used after adjusting the HLB to match the emulsified oil. In the case of using a single polyglycerol fatty acid ester or if a polyglycerol fatty acid ester having an HLB value outside the aforementioned values is used in combination, an emulsion is produced if the overall HLB of the system is within the specified range. However, an emulsion having favorable emulsification stability or hardness stability over a wide range of temperatures cannot be obtained. In addition, an emulsion can also not be obtained that has favorable particle diameter and transparency, and emulsification stability with the passage of timewith the passage of time.

The following provides a detailed explanation of each of the polyglycerol fatty acid esters used in the present invention. The highly hydrophilic polyglycerol fatty acid ester used in the present invention is required to have an HLB value of 13 or more, and preferably within the range of 13 to 17. If the HLB value is lower than 13, it becomes difficult to adjust the HLB value due to difficulty in phase inversion emulsification, thereby making it difficult to produce a satisfactory emulsion, In addition, a fine emulsion can also not be produced, and emulsification stability and hardness stability at temperatures higher than 40°C are unable to be obtained. The composite polyglycerol is required to have an average degree of polymerization of 8 to 12, and preferably 10 to 12. If the average degree of polymerization is lower than 8, it becomes difficult to adjust the HLB value due to the low level of hydrophilic properties. Polyglycerol having an HLB value larger than 12 are unable to produce a satisfactory emulsion, and since various problems occur such as poor fineness, difficulty in generating hardness, large average particle diameter and poor high-temperature stability, such a polyglycerol is not suitable for the present invention. In addition, it is also required that the total content of pentamer and hexamer be 50% by weight or more, and preferably 70% by weight or more. If the total content is less than 50% by weight, emulsification stability and maintenance of hardness at high temperatures become poor, pH decreases and other problems occur. In addition, the content of tetramer and lower polymers is required to be less than 20% by weight, and preferably 10% by weight or less. As a result of using a component polyglycerol as described above, a satisfactory emulsion can be produced having emulsification stability and hardness stability over a wide temperature range.

In the method described above, when obtaining polyglycerol having for its main component pentamer and hexamer glycerol compounds, the polyglycerol typically contains 0% to 30% by weight of heptamer and higher polymers as auxiliary components, and there presence to a certain extent does not present a problem in the present invention as well. However, in the case glycerol compounds in the form of heptamers or larger polymers are contained as the main component, a satisfactory emulsion is not produced, satisfactory fineness, hardness, high-temperature stability and emulsification stability are not obtained, the pH decreases and other problems occur, thereby making such glycerol compounds unsuitable for the present invention.

Although there are several methods for obtaining the polyglycerol of the present invention, a synthesis method in which the reaction process is controlled so that components having a certain fixed degree of polymerization are produced by using as starting substances glycidol, epichlorohydrin, monochlorohydrin, dichlorohydrin, and so forth (Japanese Unexamined Patent Application, First Publication No. 7-100355), or a column fractionation method (Japanese Unexamined Patent Application, First Publication No. 8-143513) is used preferably.

The following indicates a first aspect of the present invention.

The fatty acid residue that composes the polyglycerol fatty acid ester is required to be a linear alkyl group having 14 to 22 carbon atoms, and is preferably a linear alkyl group having 16 to 18 carbon atoms. As a result of being a linear alkyl group having 14 to 22 carbon atoms, a satisfactory emulsion can be produced that has emulsification stability and hardness stability over a wide temperature range. Typical examples of such alkyl groups include myristic acid, palmitic acid, stearic acid and behenic acid. Since alkyl groups having less than 14 carbon atoms such as lauric acid and capric acid do not have emulsification stability at temperatures higher than 40°C and exhibit decreases in hardness, they do not comply with the object of the present invention. In addition, since unsaturated fatty acids and branched fatty acids such as oleic acid and isostearic acid inhibit the hardness of the composition and do not have stability at temperatures higher than 40°C, they also do not comply with the object of the present invention. The containing of small amounts of other fatty acids for the purpose of finely adjusting functionality does not present a problem provided they do not affect the first object of the present invention.

With respect to esterification, since the polyglycerol is obtained by ordinary methods and the HLB value required in the first aspect of the present invention is 13 or more, about 8% to 15% by weight of the hydroxyl groups of the polyglycerol should be esterified.

The lipophilic polyglycerol fatty acid ester used in the first aspect of the present invention is required to have an HLB value of 5 to 11, and preferably 8 to 11. If the HLB value is outside this range, phase inversion emulsification becomes difficult, and it becomes difficult to form a satisfactory emulsion. In addition, a fine emulsion is not formed, and emulsification stability and hardness stability are unable to be obtained at high temperatures above 40°C.

The average degree of polymerization of the polyglycerol that composes the polyglycerol fatty acid ester is 2 to 12, preferably 4 to 12, and more preferably 6 to 12. In this polyglycerol, the total content of tetramer and lower polymers is 20% by weight or more. In addition, when the average degree of polymerization is taken to be n (n is 2 or more), each of (2/3)n components being contained in an amount of at least 5% by weight or more, (when (2/3)n is not a natural number, (2/3)n is considered to be a lowest natural number greater than (2/3)n), each of at least (1/3)n components being contained preferably in an amount of at least 10% by weight or more, (when (1/3)n is not a natural number, (1/3)n is considered to be a lowest natural number greater than (1/3)n), each of at least (1/2)n components being contained in an amount of at least 8% by weight or more, (when (1/2)n is not a natural number, (1/2)n is considered to be a lowest natural number greater than (1/2)n), and each of (n-1) components being contained in an amount of at least 3% by weight or more. Moreover, each of the largest degree of polymerization components being contained in an amount of 50% by weight or less, and preferably 30% by weight or less. In general, a method in which the product is obtained by a dehydration condensation reaction or a method that uses glycidol, epichlorohydrin, monochlorohydrin, dichlorohydrin, or so forth as a starting material is suitable, but the reaction method is not limited to this. Although products having a low degree of polymerization are intentionally reduced according to the reaction conditions and the use of a multistage reaction in the case of these reaction methods, thereby resulting in the production of polyglycerol having a narrow distribution of the degree of polymerization, such polyglycerol is not suitable.

In addition, the fatty acid residue that composes the polyglycerol fatty acid ester is required to be a linear alkyl group having 14 to 22 carbon atoms, and preferably a linear alkyl group having 16 to 18 carbon atoms. Typical examples of such alkyl groups include myristic acid, palmitic acid, stearic acid, arachidic acid, and behenic acid. Alkyl groups having less than 14 carbon atoms such as lauric acid and capric acid do not comply with the first object of the present invention since they do not have emulsification stability at high temperatures above 40°C and exhibit decreases in hardness. In addition, unsaturated fatty acids and branched fatty acids such as oleic acid and isostearic acid do not generate hardness and do not have stability at temperatures higher than 40°C, thereby preventing them from comply with the first object of the present invention. These fatty acids may be contained in small amounts provided they do not have an effect on the first object of the present invention.

With respect to esterification, since the polyglycerol is obtained by ordinary methods and the HLB value required in the first aspect of the present invention is 5 to 11, it is appropriate that about 15% to 70% by weight of the hydroxyl groups of the polyglycerol be esterified.

The ratio between polyglycerol fatty acid ester having an HLB value of 13 or more and polyglycerol fatty acid ester having an HLB value of 5 to 11 is preferably 20:1 to 1:4, and particularly preferably 10:1 to 1:2. The total blended amount polyglycerol fatty acid ester in the form of hydrophilic surfactant (A) in the cosmetic emulsion preparation and agent for external use of the first aspect of the present invention, having an HLB value of 13 or more, an average degree of polymerization of the polyglycerol of 8 to 12, a total content of pentamer and hexamer of 50% by weight or more, a total content of tetramer and lower polymers of less than 20% by weight, and a fatty acid residue in the form of a linear alkyl group having 14 to 22 carbon atoms, and polyglycerol fatty acid ester in the form of lipophilic surfactant (B), having an HLB value of 5 to 11, an average degree of polymerization of the polyglycerol of 2 to 12, a total content of tetramer and lower polymers of 20% by weight or more, each of (2/3)n components being contained in an amount of at least 5% by weight or more, where n (n is 2 or more) is taken to be an average degree of polymerization (when (2/3)n is not a natural number, (2/3)n is considered to be a lowest natural number greater than (2/3)n), and a fatty acid residue in the form of a linear alkyl group having 14 to 22 carbon atoms, is preferably 0.1% to 10% by weight, and particularly preferably 0.2% to 8% by weight.

Specific examples of saturated linear alcohols having 16 to 22 carbon atoms used in the first aspect of the present invention include cetanol, stearyl alcohol, cetostearyl alcohol, and behenyl alcohol. In addition, examples of linear monoalkyl glyceryl ethers include monocetyl glyceryl ether (chimyl alcohol), monostearyl glyceryl ether (batyl alcohol), and monobehenyl glyceryl ether, and these may either be used alone or as a mixture. If the number of carbon atoms is less than 16, the effect of generating hardness diminishes and hardness also decreases due to the absence of emulsification stability at high. temperatures above 40°C. In addition, if the number of carbons exceeds 22, texture becomes extremely poor and product value decreases to the extent that the product is not worth using. The blended amount of (C) saturated linear alcohol having 16 to 22 carbon atoms and/or linear monoalkyl glyceryl ether having 16 to 22 carbon atoms in the cosmetic emulsion preparation and agent for external use of the first aspect of the present invention is preferably 0.1% to 5% by weight, and particularly preferably 0.2% to 4% by weight.

The cosmetic emulsion preparation and agent for external use of the first aspect of the present invention may be blended with an oily ingredient, and any oily ingredient may be used provided it is ordinarily used in a cosmetic emulsion preparation and agent for external use. Examples of such oily agents include naturally-occurring animal and plant oils, semi-synthetic oil, hydrocarbon oils, higher fatty acids, ester oils, silicone oils, plant, animal or synthetic purified oil components, and oil-soluble vitamins.

The following provides a list of specific examples.

Examples of naturally-occurring animal and plant oils as well as semi-synthetic oils include avocado oil, linseed oil, almond oil, olive oil, carnauba wax, candelilla wax, beef tallow, beef leg tallow, beef bone tallow, hardened beef tallow, wheat germ oil, sesame oil, rice germ oil, rice bran oil, safflower oil, soybean oil, camellia oil, evening primrose oil, corn oil, rapeseed oil, horse tallow, palm oil, palm kernel oil, castor oil, hardened castor oil, sunflower oil, jojoba oil, macadamia nut oil, beeswax, mink oil, cottonseed oil, coconut oil, hardened coconut oil, peanut oil, lanolin, liquid lanolin, reduced lanolin, and lanolin isopropyl fatty acid.

Examples of hydrocarbons include squalane, squalene, ceresin, paraffin, paraffin wax, liquid paraffin, microcrystalline wax, and Vaseline.

Examples of higher fatty acids include lauric acid, myristic acid, palmitic acid, stearic acid, behenic acid, undecylenic acid, oleic acid, linoleic acid, linolenic acid, isostearic acid, and 12-hydroxystearic acid.

Examples of ester oils include diisobutyl adipate, 2-hexyldecyl adipate, di-2-heptylundecyl adipate, isostearyl isostearate, trimethylolpropane triisostearate, cetyl 2-ethylhexanoate, neopentyl glycol di-2-ethylhexanoate, trimethylol propane tri-2-ethylhexanoate, pentaerythritol tetra-2-ethylhexanoate, cetyl octanoate, oleyl oleate, octyldodecyl oleate, decyl oleate, neopentyl glycol dicaprate, 2-ethylhexyl succinate, isocetyl stearate, butyl stearate, diisopropyl sebacate, cetyl lactate, myristyl lactate, 2-ethylhexyl palmitate, cholesteryl 12-hydroxystearate, phytosteryl oleate, diisostearyl malate, paramethoxy cinnamate, and pentaerythrite tetrarosinate.

Examples of glyceride oils include glyceryl triisostearate, glyceryl triisopalmitate, glyceryl tri-2-ethylhexanoate, glyceryl trimyristate, and glyceryl diparamethoxycinnamate monoisooctylate.

Examples of silicone oils include dimethylpolysiloxane, methylphenylpolysiloxane, methylhydrogenpolysiloxane, octamethylcyclopentasiloxane, dodecamethylcyclohexasiloxane, higher alkoxy-modified silicones such as stearoxysilicone, alkyl-modified silicones, and higher fatty acid ester-modified silicones.

Examples of fat-soluble vitamins include tocopherol and its derivatives, and retinal and its derivatives.

Although examples of oily ingredients are described above, the oily ingredients used in the first aspect of the present invention are not limited to these examples. The use of an oily ingredient having for its main component (containing 50% by weight or more) a hydrocarbon oil is particularly preferable. Although an oily component is not essential, it is normally used at 100 times or less the total amount of polyglycerol fatty acid ester.

In addition, with respect to emulsification stability and hardness stability at high temperatures above 40°C that is an object of the present invention, stability is set particularly in the vicinity of 45°C, and emulsification stability and hardness stability at even higher temperatures can be achieved by blending saturated lecithin. More specifically, in the case of desiring to set emulsification stability and hardness stability to 50°C, although it is suitable to blend lecithin (saturated lecithin) within the range of 20:1 to 1:1 with respect to the total weight of polyglycerol fatty acid ester blended as emulsifier, the amount of lecithin is not specified to be this blended amount. Examples of saturated lecithin that can be used include soybean lecithin and egg yolk lecithin that can normally be obtained in the form of commercially available products and reagents, as well as purified lecithin and fractionated lecithin obtained by the solvent separation, extraction, fractionation, or so forth. Moreover, lecithin having an enhanced degree of saturation as a result of hydrogenation are particularly preferable. They are primarily composed of saturated, linear dialkyloyl phosphatidyl choline having 12 to 22 carbon atoms, dialkyloyl phosphatidyl ethanol amine and dialkyloyl phosphatidyl inositol. In addition, lyzolecithin can also be used since it exhibits similar effects.

In addition, examples of other substances that exhibit similar effects to lecithin are sterol esters. The constituent components of sterol esters are divided into sterols and fatty acid. Examples of sterols include cholesterol and phytosterol, while examples of esters include esters of oleic acid, palmitoleic acid, ricinolic acid, stearic acid, palmitic acid, myristic acid, lauric acid, and 12-hydroxystearic acid, and there are no restrictions on their sources.

The cosmetic emulsion preparation and agent for external use of the first aspect of the present invention can be blended with known ingredients used in those fields within a range that does not impair the characteristics of the present invention. Examples of substances that can be blended include aqueous ingredients such as disodium edetate; powdered ingredients such as talc, silica, kaolin, and magnesium oxide; moisturizing ingredients such as sorbitol, propylene glycol, lactic acid, and polyethylene glycol: thickeners such as carboxyvinyl polymer, carboxymethyl cellulose, polyvinyl alcohol, and carageenan; pH adjusters such as lactic acid-sodium lactate, and citric acid-sodium citrate; antioxidants such as butylhydroxytoluene and sodium hydrogen sulfite; antiseptics such as methylparaben and sodium benzoate; and ultraviolet protectants such as paraaminobenzoic acid and octylcinnamate.

The cosmetic emulsion preparation and agent for external use of the first aspect of the present invention can be produced by, for example, mixing, stirring, and dissolving the aforementioned components (A), (B), and (C), and an oily ingredient as necessary, followed by mixing therein water or other ingredients as necessary to carry out phase inversion emulsification. The temperature during phase inversion emulsification is preferably 40 to 90°C, and particularly preferably 50 to 80°C.

Here, in organizing the way of thinking regarding decreases in hardness, although hardness generally decreases when the temperature is raised in the case of creams and moisturizers that use a higher alcohol are known to allow the obtaining of hardness due to the formation of liquid crystal with surfactant in which the higher alcohol is present. The internal structure of the liquid crystal varies with temperature, and the structure ends up disappearing when the temperature becomes higher, thereby resulting in a rapid decrease in hardness. However, according to the blending of the first aspect of the present invention, although not fully understood, it is difficult for a decrease in hardness to occur for reasons such as an increase in interface density or the interface becoming rigid. In addition, with respect to evaluation of hardness, hardness is evaluated in terms of viscosity if it can be measured with a type B viscometer, and in the case of greater hardness, a method that measures consistency by a penetrometer is suitable.

Next, the following indicates a second aspect of the present invention. Furthermore, an explanation of those constituents that are the same as those of the first aspect is omitted.

The fatty acid residue that composes the polyglycerol fatty acid ester in the second aspect of the present invention is a hydrocarbon group having 14 to 22 carbon atoms, and examples of this hydrocarbon group include a linear alkyl group, branched alkyl group and alkenyl group. Typical examples of these hydrocarbon groups include myristic acid, palmitic acid, stearic acid, behenic acid, palmitoleic acid, oleic acid, isopalmitic acid, and isostearic acid. Since hydrocarbon groups having less than 14 carbon atoms such as lauric acid and capric acid have poor emulsification stability and end up clouding with the passage of time, they do not comply with the second object of the present invention. The containing of other fatty acids for the purpose of finely adjusting functionality does not present a problem provided they do not affect the second object of the present invention.

The lipophilic polyglycerol fatty acid ester used in the present invention is required to have an HLB value of 5 to 11, and preferably 8 to 11. If the HLB value is outside this range, emulsification stability becomes poor and it ends up clouding with the passage of time.

In addition, the fatty acid residue that composes the polyglycerol fatty acid ester is a hydrocarbon group having 14 to 22 carbon atoms, and preferable examples of such hydrocarbon groups include linear alkyl groups, branched alkyl groups and alkenyl groups. Specific examples of such hydrocarbon groups include myristic acid, palmitic acid, stearic acid, behenic acid, palmitoleic acid, oleic acid, isopalmitic acid, and isostearic acid. Since hydrocarbon groups having less than 14 carbon atoms such as capric acid and lauric acid exhibit decreases in permeability with the passage of time or have poor emulsification stability due to the particle diameter of the emulsion being unable to be made small, they do not comply with the second object of the present invention. Other fatty acids may be contained provided they do not affect the second object of the present invention.

In addition, the weight ratio of polyglycerol fatty acid ester having an HLB value of 13 or more and polyglycerol fatty acid ester having an HLB value of 5 to 10 is preferably 20:1 to 1:4, and particularly preferably 10:1 to 1:2. The total blended amount polyglycerol fatty acid ester in the form of hydrophilic surfactant (D) in the cosmetic emulsion preparation and agent for external use of the second aspect of the present invention, having an HLB value of 13 or more, an average degree of polymerization of the polyglycerol of 8 to 12, a total content of pentamer and hexamer of 50% by weight or more, a total content of tetramer and lower polymers of less than 20% by weight, and a fatty acid residue in the form of a hydrocarbon group having 14 to 22 carbon atoms, and polyglycerol fatty acid ester in the form of lipophilic surfactant (E), having an HLB value of 5-11, an average degree of polymerization of the polyglycerol of 2 to 12, a total content of tetramer and lower polymers of 20% by weight or more, each of (2/3)n components being contained in an amount of at least 5% by weight or more, where n (n is 2 or more) is taken to be an average degree of polymerization (when (2/3)n is not a natural number, (2/3)n is considered to be a lowest natural number greater than (2/3)n), and a fatty acid residue in the form of a hydrocarbon group having 14 to 22 carbon atoms, is preferably 0.1% to 10% by weight, and particularly preferably 0.2% to 8% by weight.

In addition, soybean lecithin and egg yolk lecithin that can normally be obtained in the form of commercially available products and reagents, as well as purified lecithin and fractionated lecithin obtained by the solvent separation, extraction, fractionation, or so forth, are used for the lecithin (component (F)). In the second aspect in particular, in consideration of oxidation stability, lecithin having an enhanced degree of saturation as a result of hydrogenation is suitable. Lecithin is particularly preferable in which the phosphatidyl choline concentration in the phospholipid component is 50% by weight or more, and particularly that in which the alkyloyl phosphatidyl choline concentration is 50% by weight or more, and the phosphatidyl inositol concentration is 3% or less, and particularly that in which the alkyloyl phosphatidyl inositol concentration is below the detection limit, on the basis of the phosphorous content as determined by thin layer chromatography (TLC). Although these can generally be obtained by treatment consisting of alcohol extraction and column fractionation, they may be obtained by other methods as well. Although those obtained by chemical synthesis can also be used, when considering in terms of cost, those extracted from natural substances are suitable. In addition, lyzolecithin obtained by enzyme conversion and chemical synthesis is also within this range.

The ratio of lecithin and polyglycerol fatty acid ester is preferably 1:20 to 4:1, and particularly preferably 1:15 to 3:1. The content of lecithin in the transparent cosmetic emulsion preparation and agent for external use of the second aspect of the present invention is preferably 0.1% to 10% by weight, and particularly preferably 0.2% to 8% by weight.

An oily ingredient (G) is used in the transparent cosmetic emulsion preparation and agent for external use of the second aspect of the present invention. Although any oily ingredient may be used provided it is typically used in cosmetics and agents for external use, an oily ingredient that is a liquid or paste at normal temperatures is particularly suitable. The oily ingredient of the second aspect of the present invention is that which is obtained by removing the naturally-occurring animal or plant oils and semi-synthetic oils such as carnauba wax, candelilla wax, beef leg tallow, beef bone tallow, hardened beef tallow, hardened castor oil, beeswax, or hardened coconut oil; hydrocarbon oils such as ceresin, paraffin, paraffin wax, microcrystalline wax, and long-chained fatty acid esters from the oily ingredient of the first aspect of the present invention followed by the addition of hydrocarbon oil in the form of olefin oligomer.

In addition, the weight ratio of the total amount of polyglycerol fatty acid ester and lecithin to the amount of oily component is preferably 100:1 to 1:2, and in the absence of the oily component, emulsification stability and pH stability are impaired. The blended amount of oily ingredient is preferably 0.01% to 10% by weight.

Oily ingredients that are particularly effective in stabilizing emulsification are those in which the fatty acid residue is a sterol ester having 14 to 22 carbon atoms, and is preferably contained in the oily ingredient. The constituent components of sterol esters are as indicated in the first aspect of the present invention.

Furthermore, pH stability with the passage of time is correlated with emulsification stability, and in cases in which emulsification stability is satisfactory, pH is resistant to decreases, while in the case of poor emulsification stability, pH tends to decrease. However, according to the blend of the second aspect of the present invention, although not fully understood, substrate of a hydrolysis reaction is presumed to become increasing resistant to attack at an ester site for reasons such as an increase in interface density or the interface becoming rigid.

In addition, the transparent cosmetic emulsion preparation and agent for external use of the second aspect of the present invention is required to have an average particle diameter of the emulsion of 100 nm or less, and preferably 80 nm or less. If the average particle diameter is 100 nm or less, a transparent cosmetic emulsion preparation and agent for external use are obtained having superior emulsification stability and pH stability without a decrease in transparency or separation with the passage of time. An emulsion having such an average particle diameter can be obtained by adjusting the ratio of hydrophilic polyglycerol fatty acid ester, lipophilic polyglycerol fatty acid ester, and lecithin according to the vicinity of the required HLB of the oily ingredient. Furthermore, average particle diameter is measured using laser scattering spectroscopy. Furthermore, the transparent cosmetic referred to here indicates that having transmittance of 50% T or more, and preferably 70% T or more, using purified water as a control and measuring with a spectrophotometer when the measurement wavelength is 750 nm and the measurement light path is 10 mm.

The transparent cosmetic emulsion preparation and agent for external use of the second aspect of the present invention can be produced by, for example, mixing, stirring and emulsifying the aforementioned components (D), (E), (F), and (G), followed by mixing, stirring and emulsifying therein water or other ingredients as necessary. The temperature at this time is preferably 40 to 90°C, and particularly preferably 50 to 80°C. In addition, the average particle diameter can be made to be 100 nm or less by suitably selecting the stirring conditions and so forth at this time, or suitably adjusting the HLB values of both polyglycerol fatty acid esters.

In addition, the transparent cosmetic emulsion preparation and agent for external use of the second aspect of the present invention can also be applied to products not containing an antiseptic as an added ingredient from the perspective of safety. In the case of containing one or a combination of two or more of either of 1,3-butylene glycol, 3-methyl-1,3-butylene glycol, 1,2-pentanediol, propylene glycol, or 1,2-hexanediol, or in the case of preparing with an ethylene oxide-based surfactant, even if a transparent composition is obtained by a fine emulsion, when they are blended at a high concentration of, for example, 15% to 25% by weight, there was the problem of clouding occurring with the passage of time. According to the present invention, even if these are blended, a transparent cosmetic and agent for external use that has stable emulsification.

There are no particular restrictions on the form of the cosmetic emulsion preparation and agent for external use of the present invention, and may be in any arbitrary form such as a cream, moisturizer, essence, ointment, gel, lotion, or pack.

The cosmetic emulsion preparation and agent for external use of the present invention can be used according to the ordinary methods of use of each form.

The cosmetic emulsion preparation and agent for external use of the present invention may be blended with known ingredients ordinarily used in those fields provided they are within a range that does not impair the characteristics of the present invention.

### Examples

The following provides a detailed explanation of the present invention through its examples, comparative examples and reference examples, however, these are provided for the purpose of demonstrating examples of the present invention, and do not limit the present invention in any way.

### Production Example 1 - Polyglycerol

3300 g of diglycerol and 800 g of a 50% aqueous sodium hydroxide solution were placed in a 5-liter four-mouth flask and heated to 140°C while removing the water in the presence of flowing nitrogen. After distillation of the water was completed, 640 g of dichlorohydrin were added dropwise over 2 hours. Following adding dropwise, the mixture was stirred for 2 hours at 120°C. After removing the excess diglycerol by molecular distillation, the mixture was diluted with water, and decolored and desalted with activated charcoal and ion exchange resin followed by removal of water to obtain polyglycerol. When the hydroxyl value of this product was measured and the average degree of polymerization was calculated, it was found to be 10. In addition, when this product was treated with TMS and analyzed according to the aforementioned GC method, the weight percentages were found to be 0% for free glycerol, 2% for the dimer component, 3% for the trimer component, 5% for the tetramer component, 60% for the pentamer component, 15% for the hexamer component, 5% for the heptamer component, 4% for the octamer component, 3% for the nonamer component, 1% for the decamer component, and 2% for the undecamer and higher polymer component.

### Production Example 2 - Polyglycerol

1000 g of the polyglycerol of Production Example 1 were applied to a pseudo moving bed chromatography system composed of four columns having a degree of crosslinking of 12% filled with 10 ml of calcium cation exchange resin, and then eluted with water. The ratio of the supply and outflow time to the circulation time was set at 2:1. The outflow liquid was separated into polyglycerol having a degree of polymerization of 3 or more while measuring with a refractometer, and then dehydrated and concentrated with a rotary evaporator to obtain polyglycerol. The same procedure was carried out four times using the same column. The resulting polyglycerol had an average degree of polymerization of 11, and its composition consisted of 1% free glycerol, 1% dimer component, 2% trimer component, 6% tetramer component, 58% pentamer component, 18% hexamer component, 4% octamer component, 2% nonamer component, 2% decamer component and 3% undecamer and higher polymer component.

### (1) Synthesis Examples of Polyglycerol Fatty Acid Ester Having a High HLB Value (Component (A) or (D))

### Synthesis Example 1 - Polyglycerol Fatty Acid Ester

259.7 g of the polyglycerol obtained in Production Example 1, 88.2 g of stearic acid and 0.1 g of tripotassium phosphate were placed in a 500 ml four-mouth flask and allowed to react at 250°C while removing the water produced in the presence of flowing nitrogen. After the reaction, 0.3 ml of phosphoric acid were added to obtain polyglycerol stearate. The acid value of this ester was 1.0. The HLB value of the polyglycerol stearate was 13.4.

### Synthesis Example 2 - Polyglycerol Fatty Acid Ester

Polyglycerol stearate was obtained by the same process as Synthesis Example 1 with the exception of using the polyglycerol obtained in Production Example 2 instead of the polyglycerol obtained in Production Example 1. The acid value of this ester was 1.0. The HLB value of the polyglycerol stearate was 13.9.

### Synthesis Example 3 - Polyglycerol Fatty Acid Ester

Polyglycerol myristate was obtained by the same process as Synthesis Example 1 from 269.5 g of the polyglycerol obtained in Production Example 1 and 80.5 g of myristic acid. The acid value of this ester was 1.0. The HLB value of this polyglycerol myristate was 15.0.

### Synthesis Example 4 - Polyglycerol Fatty Acid Ester

Polyglycerol oleate was obtained by the same process as Synthesis Example 1 from 261.6 g of the polyglycerol obtained in Production Example 1 and 85.6 g of oleic acid. The acid value of this ester was 1.0. The HLB value of the polyglycerol oleate was 13.0.

### Synthesis Comparative Example 1 - Polyglycerol Fatty Acid Ester

Polyglycerol isostearate was obtained by the same process as Synthesis Example 1 from 210 g of the polyglycerol obtained in Production Example 1 and 90 g of isostearic acid. The acid value of this ester was 1.2. The HLB value of the polyglycerol isostearate was 13.0.

### Synthesis Comparative Example 2 - Polyglycerol Fatty Acid Ester

Polyglycerol laureate was obtained by the same process as Synthesis Example 1 from 210 g of the polyglycerol obtained in Production Example 2 and 52.5 g of lauric acid. The acid value of this ester was 1.0. The HLB value of the polyglycerol laureate was 15.6.

### Synthesis Comparative Example 3 - Polyglycerol Fatty Acid Ester

Polyglycerol stearate was obtained by the same process as Synthesis Example 1 with the exception of using Grade Oil #1000 (Taiyo Kagaku Co., Ltd.) (average degree of polymerization: 10, free glycerin: 8%, dimer component: 20%, trimer component: 20%, tetramer component: 13%, pentamer component: 10%, hexamer component: 7%, heptamer component: 8%, octamer component: 6%, nonamer component: 4%, decamer component: 1%, undecamer and higher polymer component: 3%) instead of the polyglycerol obtained in Production Example 1. The HLB value was 12.0.

### Synthesis Comparative Example 4 - Polyglycerol Fatty Acid Ester

Polyglycerol myristate was obtained by the same process as Synthesis Example 3 with the exception of using Grade Oil #1000 (Taiyo Kagaku Co., Ltd.) instead of the polyglycerol obtained in Production Example 1. The acid value of this ester was 1.0. The HLB value was 14.0.

### Synthesis Comparative Example 5 - Polyglycerol Fatty Acid Ester

Polyglycerol laureate was obtained by the same process as Synthesis Example 1 from 210 g of the polyglycerol obtained in Production Example 2 and 52.5 g of lauric acid. The acid value of this ester was 1.0. The HLB value of thepolyglycerol laureate was 15.6.

### (2) Synthesis Examples of Polyglycerol Fatty Acid Esters Having a Low HLB Value (Component (B) or (E))

### Synthesis Example 5 - Polyglycerol Fatty Acid Ester

164.5 g of Grade Oil #1000 (Taiyo Kagaku, Co., Ltd.), 185.5 g of stearic acid and 0.1 g of tripotassium phosphate were placed in a 500 ml four-mouth flask and allowed to react at 250°C while removing the water produced in the presence of flowing nitrogen. After the reaction, 0.3 ml of phosphoric acid were added to obtain polyglycerol stearate. The acid value of this ester was 1.5. The HLB value of the polyglycerol stearate was 9.0.

### Synthesis Example 6 - Polyglycerol Fatty Acid Ester

Polyglycerol palmitate was obtained by the same process as Synthesis Example 5 from 175.0 g of Grade Oil #1000 (Taiyo Kagaku, Co., Ltd.) and 175.0 g of palmitic acid. The acid value of this ester was 1.5. The HLB value of the polyglycerol palmitate was 9.5.

### Synthesis Example 7 - Polyglycerol Fatty Acid Ester

Polyglycerol stearate was obtained by the same process as Synthesis Example 5 from 115.5 g of Grade Oil #600 (Taiyo Kagaku., Co., Ltd.) (average degree of polymerization: 6, free glycerin: 0%, dimer component: 6%, trimer component: 24%, tetramer component: 22%, pentamer component: 15%, hexamer component: 10%, heptamer component: 10%, octamer component: 8%, nonamer component: 5%, decamer component: 0%, undecamer and higher polymer component: 0%) and 234.5 g of stearic acid. The acid value of this ester was 0.5. The HLB value of the polyglycerol stearate was 6.0.

### Synthesis Example 8 - Polyglycerol Fatty Acid Ester

Polyglycerol oleate was obtained by the same process as Synthesis Example 5 from 168.0 g of Grade Oil #1000 (Taiyo Kagaku, Co., Ltd.) and 182.0 g of oleic acid. The acid value of this ester was 1.0. The HLB value of the polyglycerol oleate was 9.2.

### Synthesis Comparative Example 6 - Polyglycerol Fatty Acid Ester

Polyglycerol stearate was obtained by the same process as Synthesis Example 5 with the exception of using the polyglycerol of Production Example 1 instead of Grade Oil #1000 (Taiyo Kagaku, Co., Ltd.). The HLB value was 9.5.

### Synthesis Comparative Example 7 - Polyglycerol Fatty Acid Ester

Polyglycerol oleate was obtained by the same process as Synthesis Example 5 from 168.0 g of Grade Oil #1000 (Taiyo Kagaku, Co., Ltd.) and 182.0 g of oleic acid. The acid value of this ester was 1.0. The HLB value of the polyglycerol oleate was 9.2.

### Synthesis Comparative Example 8 - Polyglycerol Fatty Acid Ester

Polyglycerol stearate was obtained by the same process as Synthesis Example 5 from 199.5 g of Grade Oil #1000 (Taiyo Kagaku, Co., Ltd.) and 150.5 g of stearic acid. The acid value of this ester was 1.0. The HLB value of the polyglycerol stearate was 11.6.

### Synthesis Comparative Example 9 - Polyglycerol Fatty Acid Ester

Polyglycerol stearate was obtained by the same process as Synthesis Example 5 from 87.5 g of Grade Oil #1000 (Taiyo Kagaku, Co., Ltd.) and 262.5 g of stearic acid. The acid value of this ester was 1.5. The HLB value of the polyglycerol stearate was 4.0.

### Synthesis Comparative Example 10 - Polyglycerol Fatty Acid Ester

Polyglycerol laureate was obtained by the same process as Synthesis Example 5 from 170.0 g of Grade Oil #1000 (Taiyo Kagaku, Co., Ltd.) and 80.0 g of lauric acid. The acid value of this ester was 1.5. The HLB value of the polyglycerol laureate was 9.5.

### Examples 1 through 8 and Comparative Examples 1 through 12

Cosmetic emulsion preparations (creams) were prepared according to the blends indicated in Tables 1 and 2. Namely, polyglycerol fatty acid ester, higher alcohol and an oily ingredient were mixed and dissolved while stirring with a homomixer (3000 rpm) at 70°C followed by pouring 70°C water or a mixed solution of water and 1,3-butylene glycol, and other ingredients to carry out phase inversion emulsification. Subsequently, aqueous sodium hydroxide solution was added followed by the addition of "CARBOPOL®" aqueous solution. The emulsion was cooled to 30°C, removed and then allowed to stand undisturbed for 24 hours at 25°C to obtain a cream. Furthermore, the blending ratio of hydrophilic polyglycerol fatty acid ester and lipophilic polyglycerol fatty acid ester was selected that resulted in the minimum average particle diameter so as to match the HLB (excluding Comparative Example 7 in which only a hydrophilic polyglycerol fatty acid ester was used, and Comparative Example 11 due to the low HLB value of the hydrophilic polyglycerol fatty acid ester). The average particle diameter was measured using the LA-500 Laser Refraction Particle Size Distribution Measuring System (manufactured by Horiba Ltd.).

Each of the creams obtained above were evaluated for the following parameters (1) through (5). Those results are shown in Tables 1 and 2.
(1) Emulsification stability: Oil separation and creaming were checked after storing for 1 month at 45°C. Stable: ○, Creaming: Δ, Oil phase separation:
(2) Change in hardness: Viscosity at 25°C and 45°C immediately after production was measured with a BL viscometer. The ratio of each viscosity was calculated and evaluated using the following standards.
(45°C viscosity)/(25°C viscosity)
2/3 or more: ⓞ
1/2 to less than 2/3: ○
1/3 to less than 1/2: Δ
Less than 1/3:

(3) Low-temperature stability: Viscosity at 25°C was measured after storing for 1 month at 0°C. The ratio of the viscosity at 25°C immediately after production and the viscosity at 25°C after storage for 1 month at 0°C was evaluated using the following standards.
(Viscosity after 1 month)/(Viscosity immediately after)
9/10 to less than 11/10: ⓞ
11/10 to less than 6/5: ○
6/5 to less than 3/2: Δ
Less than 3/2:

(4) Appearance: Texture was assessed visually.
Extremely good texture: ⓞ
Good texture: ○
Somewhat poor texture: Δ
Poor texture:

(5) Vanishing during use: A product was applied to the upper arm in about the size of a cherry and vanishing was evaluated by using a stopwatch to measure the amount of time until it smoothly disappeared.
Disappeared in less than 3 seconds: ⓞ
Disappeared in 3 to less than 5 seconds: ○
Disappeared in 5 to less than 10 seconds: Δ
Did not disappear even after 10 seconds:

**Table 1**

| Examples | 1 | 2 | 3 | 4 | 5 | 6 | 7 | 8 |
|---|---|---|---|---|---|---|---|---|
| Synthesis Example 1 | 1.6 | - | - | 2.0 | 1.4 | 1.4 | - | - |
| Synthesis Example 2 | - | 1.6 | - | - | - | - | 2.5 | 2.4 |
| Synthesis Example 3 | - | - | 1.5 | - | - | - | - | - |
| Synthesis Example 5 | 0.4 | 0.4 | 0.5 | - | - | 0.6 | - | - |
| Synthesis Example 6 | - | - | - | 1.0 | - | - | - | - |
| Synthesis Example 7 | - | - | - | - | 0.2 | - | 0.5 | 0.6 |
| Cetanol | 2 | 2 | 2 | 2 | 2 | 1 | 2 | 2 |
| Stearyl alcohol | - | - | - | - | - | 1 | 1 | 2 |
| Liquid paraffin | 20 | 20 | 20 | 20 | 20 | 10 | 20 | 20 |
| Glyceryl tri-2-ethyl-hexanoate | - | - | - | - | - | 10 | 10 | 10 |
| 1,3-butylene glycol | 10 | 10 | 10 | 10 | 10 | 15 | - | 10 |
| Purified water | 54 | 54 | 54 | 53 | 54.4 | 51 | 49.5 | 53 |
| 1% aq. carboxyvinyl polymer | 10 | 10 | 10 | 10 | 10 | 10 | 12 | - |
| 1% aq. sodium hydroxide | 2 | 2 | 2 | 2 | 2 | 2 | 2.5 | - |
| Avg. particle diameter | 0.82 | 0.75 | 0.65 | 0.51 | 0.98 | 0.78 | 0.88 | 0.95 |
| Emulsification stability | ○ | ○ | ○ | ○ | ○ | ○ | ○ | ○ |
| Viscosity after production 25°C (mPa·s) | 60400 | 54500 | 59600 | 68000 | 40500 | 72500 | 89800 | 86000 |
| Viscosity after production 45°C (mPa·s) | 39900 | 37600 | 32600 | 41000 | 22300 | 42000 | 62000 | 54400 |
| Change in hardness | ○ | ⓞ | ○ | ○ | ○ | ○ | ⓞ | ○ |
| 25°C viscosity after storing 1 month at 0°C | 60800 | 54000 | 61000 | 69800 | 40100 | 74500 | 92400 | 89000 |
| Low-temp. stability | ⓞ | ⓞ | ⓞ | ⓞ | ⓞ | ⓞ | ⓞ | ⓞ |
| Texture in appearance | ⓞ | ⓞ | ⓞ | ⓞ | ⓞ | ⓞ | ○ | ○ |
| Vanishing during use | ⓞ | ⓞ | ⓞ | ⓞ | ○ | ○ | ○ | ○ |
| ∗ Units: g | | | | | | | | |

**Table 2**

| Comp. Ex. | 1 | 2 | 3 | 4 | 5 | 6 | 7 | 8 | 9 | 10. | 11 | 12 |
|---|---|---|---|---|---|---|---|---|---|---|---|---|
| Syn. Ex. 1 | 1.6 | 1.6 | 1.4 | 1.8 | 1.8 | 1.8 | 2 | - | - | - | - | - |
| Syn. Ex. 5 | - | - | - | - | - | - | | 0.4 | 0.2 | 0.5 | 0.2 | 0.2 |
| Syn. Comp. Ex.1 | - | - | - | - | - | - | - | - | 1.8 | - | - | - |
| Syn. Comp. Ex.2 | - | - | - | - | - | - | - | - | - | 1.5 | - | - |
| Syn.Comp. Ex.3 | - | - | - | - | - | - | - | - | - | - | 1.8 | - |
| Syn. Comp. Ex.4 | - | - | - | - | - | - | - | - | - | - | - | 1.8 |
| Syn. Comp. Ex.6 | 0.4 | - | - | - | - | - | - | - | - | - | - | - |
| Syn. Comp. Ex.7 | - | 0.4 | - | - | - | - | - | - | - | - | - | - |
| Syn. Comp. Ex.8 | - | - | 0.6 | - | - | - | - | - | - | - | - | - |
| Syn. Comp. Ex.9 | - | - | - | 0.2 | - | - | - | - | - | - | - | - |
| Stearic mono-glyceride | - | - | - | - | 0.2 | - | - | - | - | - | - | - |
| Sorbitan mono-stearate | - | - | - | - | - | 0.2 | - | - | - | - | - | - |
| EO (20 mol) sorbitan mono-stearate | - | - | - | - | - | - | - | 1.6 | - | - | - | - |
| Cetanol | 2 | 2 | 3 | 1 | 2 | 1 | 2 | 2 | 2 | 2 | 2 | 2 |
| Stearyl alcohol | - | - | - | 1 | - | 1 | - | - | - | - | - | 2 |
| Liquid paraffin | 20 | 20 | 10 | 20 | 20 | 15 | - | 20 | 20 | 20 | 10 | 15 |
| Glyceryl tri-2-ethyl-hexanoate | - | - | 10 | - | 10 | 15 | 20 | - | - | - | 10 | 15 |
| 1,3-butylene glycol | 10 | 10 | 10 | 15 | 10 | 10 | 10 | 10 | - | 10 | 10 | - |
| Purified water | Rem. | Rem. | Rem. | Rem. | Rem. | Rem. | Rem. | Rem. | Rem. | Rem. | Rem. | Rem. |
| 1% aq. carboxyvinyl polymer | 10 | 10 | 10 | 10 | 10 | 10 | 12 | 10 | 10 | 10 | 10 | - |
| 1% aq. sodium hydroxide | 2 | 2 | 2 | 2 | 2 | 2 | 2.4 | 2 | 2 | 2 | 2 | - |
| Avg. particle diameter (µm) | 1.23 | 0.65 | 2.23 | 2.01 | 1.12 | 156 | 3.86 | 0.75 | 0.78 | 1.24 | 4.62 | 1.24 |
| Emulsification stability | | | Δ | Δ | | | | | | | | |
| Viscosity after production 25°C (mPa·s) | 58800 | 9800 | 54200 | 60300 | 57400 | 43600 | 64500 | 38900 | 6800 | 34500 | 56700 | 59500 |
| Viscosity after production 45°C (mPa·s) | 4440 | 2240 | 20600 | 22200 | 6430 | 4160 | 7320 | 4590 | 1240 | 4950 | 6500 | 7820 |
| Change in hardness | | | Δ | Δ | | | | | | | | |
| 25°C viscosity after storing 1 month at 0°C | 87300 | 9900 | >100000 | >100000 | 86500 | 65000 | >100000 | 39800 | 6990 | 48300 | >100000 | >100000 |
| Low-temp. stability | Δ | ⓞ | | | Δ | Δ | | ⓞ | ⓞ | ⓞ | | |
| Texture in appearance | Δ | ⓞ | Δ | Δ | Δ | Δ | | ⓞ | ⓞ | Δ | | ○ |
| Vanishing during use | Δ | ⓞ | | | Δ | Δ | | ⓞ | ⓞ | Δ | | Δ |
| ∗ Units: g | | | | | | | | | | | | |

The creams of Examples 1 through 8 exhibited superior emulsification stability, low-temperature stability, appearance and vanishing during use, and exhibited hardly any changes in hardness. In contrast, the creams of Comparative Examples 1 through 12 were inferior to the creams of Examples 1 through 8 for all parameters. As a result, the effect of the present invention was confirmed. Furthermore, Fig. 1 shows a comparison of viscosity at each temperature between the cream of Example 1 and the cream of Comparative Example 1. It can be understood from Fig. 1 that the change in viscosity with respect to temperature of the product of the present invention is low.

### Example 9 - Emollient Cream

An emollient cream was prepared according to the blend indicated below. Namely, ingredients (1) through (11) were mixed and dissolved while stirring with a homomixer (5000 rpm) at 75°C followed by pouring in a mixed solution of ingredients (12) through (14) at 75°C to carry out phase inversion emulsification. Subsequently, ingredient (15) was added followed by the addition of ingredient (16). The emulsion was then cooled to 30°C, removed and allowed to stand undisturbed for 24 hours at 25°C to obtain a cream. The cream was evaluated in compliance with Examples 1 through 8, and the average particle diameter was 0.56 ì m, emulsification stability was ○, change in hardness was ○ (viscosity: 70200 mPa·s at 25°C, 43600 mPa·s at 45°C), low-temperature stability was ⓞ (viscosity: 71000 mPa·s), texture in appearance was ⓞ, and vanishing during use was ⓞ.

| | | |
|---|---|---|
| (1) | Synthesis Example 1 | 1.0 |
| (2) | Synthesis Example 3 | 1.0 |
| (3) | Synthesis Example 5 | 0.6 |
| (4) | Synthesis Example 6 | 0.4 |
| (5) | Cetanol | 1.2 |
| (6) | Stearyl alcohol | 1.2 |
| (7) | Methylparaben | 0.1 |
| (8) | Squalane | 16.0 |
| (9) | Dicapric neopentyl glycol | 12.0 |
| (10) | Microcrystalline wax | 2.0 |
| (11) | Dimethylpolysiloxane | 0.1 |
| (12) | Propylene glycol | 12.0 |
| (13) | Glycerin | 4.0 |
| (14) | Purified water | 45.4 |
| (15) | Triethanolamine | 1.0 |
| (16) | 1% aqueous carboxyvinyl polymer solution | 10.0 |
| | Total | 100.0 g |

### Example 10 - Emollient Cream

An emollient cream was obtained in the same manner as the blend of Example 9 with the exception of replacing ingredient (4) with soybean hydrogenated phospholipid (PC concentration: 60 wt%). The cream was evaluated in compliance with Examples 1 through 8, and the average particle diameter was 0.50 ìm, emulsification stability was ○, change in hardness was ⓞ (viscosity: 74000 mPa·s at 25°C, 49600 mPa·s at 50°C), low-temperature stability was ⓞ (viscosity: 75500 mPa·s), texture in appearance was ⓞ, and vanishing during use was ⓞ.

### Example 11 - Moisturizer

A moisturizer was prepared according to the blend shown below. Namely, ingredients (1) through (11) were mixed and dissolved while stirring with a stirrer at 65°C followed by pouring in a mixed solution of ingredients (12) through (14) at 75°C to carry out phase inversion emulsification. Subsequently, ingredient (15) was added followed by the addition of ingredient (16). The emulsion was then cooled to 30°C, removed and allowed to stand undisturbed for 24 hours at 25°C to obtain a moisturizer. The moisturizer was evaluated in the same manner, the average particle diameter was 0.68 ì m, emulsification stability was ○, change in hardness was ○ (viscosity: 8620 mPa·s at 25°C, 5400 mPa·s at 45°C), low-temperature stability was ⓞ (viscosity: 8880 mPa·s at 25°C), texture in appearance was ⓞ, and vanishing during use was ⓞ.

| | | |
|---|---|---|
| (1) | Synthesis Example 1 | 0.5 |
| (2) | Synthesis Example 4 | 0.2 |
| (3) | Cetostearyl alcohol | 1.0 |
| (4) | Liquid paraffin | 2.0 |
| (5) | Macadamia nut oil | 2.0 |
| (6) | Hydroxystearic dipentaerythritol | 1.0 |
| (7) | 1,3-butylene glycol | 18.0 |
| (8) | Purified water | 75.9 |
| (9) | Xanthan gum | 0.2 |
| (10) | Hydroxypropyl methylcellulose | 0.2 |
| | Total | 100.0 g |

### Example 12 - Moisturizer

A moisturizer was obtained in the same manner as the blend of Example 11 with the exception of replacing ingredient (6) with phytosteryl oleate. The moisturizer was evaluated in compliance with Examples 1 through 8, and the average particle diameter was 0.58 ì m, emulsification stability was ○, change in hardness was ⓞ (viscosity: 9020 mPa·s at 25°C, 6360 mPa·s at 45°C), low-temperature stability was ⓞ (viscosity: 8960 mPa·s at 25°C), texture in appearance was ⓞ, and vanishing during use was ⓞ.

### Example 13 - UV Cream

A UV cream was prepared according to the blend indicated below. Namely, ingredients (1) through (11) were mixed and dissolved while stirring with a homomixer (5000 rpm) at 75°C followed by pouring in a mixed solution of ingredients (12) through (14) at 75°C to carry out phase inversion emulsification. Subsequently, ingredient (15) was added. The emulsion was then cooled to 30°C, removed and allowed to stand undisturbed for 24 hours at 25°C to obtain a cream. Although the cream was evaluated in compliance with Examples 1 through 8, since the viscosity was 100000 mPa·s or more, a BH viscometer was used. The average particle diameter was 0.88 ì m, emulsification stability was ○, change in hardness was ○ (viscosity: 244000 mPa·s at 25°C, 166000 mPa·s at 45°C), low-temperature stability was ⓞ (viscosity: 254000 mPa·s at 25°C), texture in appearance was ⓞ, and vanishing during use was ⓞ.

| | | |
|---|---|---|
| (1) | Synthesis Example 2 | 2.0 |
| (2) | Synthesis Example 4 | 0.5 |
| (3) | Synthesis Example 6 | 0.5 |
| (4) | Behenyl alcohol | 1.0 |
| (5) | Monostearyl glyceryl ether | 1.0 |
| (6) | Cetanol | 1.0 |
| (7) | Olive squalane | 16.0 |
| (8) | Tetra-2-ethylhexanoic pentaerythritol | 4.0 |
| (9) | Glyceryl diparamethoxycinnamate monoisooctylate | 4.0 |
| (10) | Parasol 1789 | 4.0 |
| (11) | Methylparaben | 0.2 |
| (12) | Glycerin | 4.0 |
| (13) | 1,3-butylene glycol | 10.0 |
| (14) | Purified water | 51.5 |
| (15) | Xanthan gum | 0.3 |
| | Total | 100.0 g |

### Example 14 - Moisturizer

A moisturizer was obtained in the same manner as the blend of Example 11 with the exception of replacing ingredient (2) with Sunsoft Q-183Y (Taiyo Kagaku) (polyglycerol stearic ester, HLB value: 10.2; component polyglycerol has an average degree of polymerization of 10, and is composed of 3% free glycerin, 5% dimer component, 7% trimer component, 8% tetramer component, 9% pentamer component, 10% hexamer component, 12% heptamer component, 21% octamer component, 12% nonamer component, 8% decamer component, and 5% undecamer component). The average particle diameter was 0.50 ì m, emulsification stability was ⓞ, change in hardness was ⓞ (viscosity: 9000 mPa·s at 25°C, 6120 mPa·s at 45°C), low-temperature stability was ⓞ (viscosity: 9040 mPa·s at 25°C), texture in appearance was ⓞ, and vanishing during use was ⓞ.

### Examples 9 through 18 and Comparative Examples 13 through 25

Lotions were prepared according to the blends shown in Tables 3 and 4. Namely, polyglycerol fatty acid ester, lecithin and an oily ingredient were mixed and dissolved while stirring with a stirrer at 70°C followed by pouring in a mixed solution of water, 1,3-butylene glycol, and other ingredients at 70°C, and then cooling to 30°C and removing the emulsion to obtain a lotion. Furthermore, the blending ratio of hydrophilic polyglycerol fatty acid ester and lipophilic polyglycerol fatty acid ester was selected that resulted in the minimum average particle diameter so as to match the HLB (excluding Comparative Example 18 in which only a hydrophilic polyglycerol fatty acid ester was used, and Comparative Example 19 due to the low HLB value of the hydrophilic polyglycerol fatty acid ester). The average particle diameter was measured with the Coulter Counter N-4. In addition, transmittance was measured with the JASCOV-570 spectrophotometer (JASCO Corporation) using purified water as a control at a measurement wavelength of 750 nm and measurement light path of 10 mm. The pH was measured with the F-22 pH Meter (Horiba Ltd.).

Each of the lotions obtained above were evaluated for the following parameters (1) through (3). Those results are shown in Tables 3 and 4.
(1) Emulsification stability: Oil separation and creaming were checked after storing for 1 month at 40°C. Stable: ○, Creaming: Δ, Oil phase separation:
(2) Transmittance: Transmittance was measured immediately after production and after storing for 1 month at 0°C, and evaluated using the following standards.
Decrease in transmittance
Less than 1%T: ⓞ
1%T to less than 3%T: ○
3%T to less than 10%T: Δ
10%T or more:

(3) pH: pH was measured immediately after production and after storing for 1 month at 0°C, and evaluated using the following standards.
Decrease in pH
Less than 0.1: ⓞ
0.1 to less than 0.2: ○
0.2 to less than 0.5: Δ
0.5 or more:

**Table 3**

| Examples | 9 | 10 | 11 | 12 | 13 | 14 | 15 | 16 | 17 | 18 |
|---|---|---|---|---|---|---|---|---|---|---|
| Syn. Ex. 1 | 1.6 | - | - | - | - | - | - | 1.6 | - | - |
| Syn. Ex. 2 | - | 1'.5 | - | - | - | - | 2.4 | - | - | - |
| Syn. Ex. 3 | - | - | 1.2 | - | 1.5 | 1.2 | - | - | 0.8 | 1.6 |
| Syn. Ex. 4 | - | - | - | 1.7 | - | - | - | - | - | - |
| Syn. Ex. 5 | 0.3 | 0.4 | 0.8 | 0.3 | - | - | - | 0.2 | - | - |
| Syn. Ex. 7 | - | - | - | - | 0.3 | - | - | - | - | - |
| Syn. Ex. 8 | - | - | - | - | - | 0.6 | 0.3 | - | 0.2 | - |
| Polyglyceryl isostearate¹⁾ | - | - | - | - | - | - | - | - | - | 0.3 |
| Hydrogenated soybean lipid²⁾ | 0.1 | 0.1 | 0.1 | 0.1 | 0.2 | 0.2 | 0.3 | 0.2 | 1.0 | 0.1 |
| Liquid paraffin | 2.0 | 2.0 | 2.0 | 2.0 | 2.0 | 2.0 | - | 3.0 | 0.1 | 1.0 |
| Octyl palmitate | - | - | - | - | - | - | 2.0 | - | - | 0.5 |
| 1,3-butylene glycol | 10.0 | 10.0 | 10.0 | 10.0 | 10.0 | 10.0 | 10.0 | 10.0 | 10.0 | 10.0 |
| Purified water | 86.0 | 86.0 | 86.0 | 86.0 | 86.0 | 86.0 | 85.0 | 85.0 | 87.9 | 86.5 |
| Avg. particle diameter (nm) | 45.0 | 48.0 | 42.0 | 43.0 | 38.0 | 41.0 | 37.0 | 66.0 | 62.0 | 35.0 |
| Emulsification stability | ○ | ○ | ○ | ○ | ○ | ○ | ○ | ○ | ○ | ○ |
| Transmittance after production (%T) | 85.0 | 82.0 | 87.3 | 86.0 | 90.5 | 88.3 | 94.2 | 78.5 | 88.0 | 96.4 |
| Transmittance after 1 month (%T) | 84.5 | 81.6 | 87.1 | 85.6 | 90.3 | 88.3 | 94.2 | 76.9 | 86.8 | 94.0 |
| Transmittance stability | ⓞ | ⓞ | ⓞ | ⓞ | ⓞ | ⓞ | ⓞ | ○ | ○ | ○ |
| pH after production | 6.12 | 6.18 | 6.32 | 6.24 | 6.19 | 6.16 | 6.17 | 6.25 | 6.07 | 6.34 |
| pH after 1 month | 6.11 | 6.13 | 6.32 | 6.17 | 6.04 | 6.03 | 6.06 | 6.18 | 5.85 | 6.10 |
| pH stability | ⓞ | ⓞ | ⓞ | ⓞ | ○ | ⓞ | ⓞ | ○ | ○ | ○ |

| | | | | | | | | | | |
|---|---|---|---|---|---|---|---|---|---|---|
| ∗ Units: g 1) Cosmol 41 (manufactured by The Nisshin Oil Mills, Ltd.), polyglycerol average degree of polymerization: 2 (free glycerin: 6%, dimer: 90%, trimer: 4%), HLB value: 5.1 | | | | | | | | | | |
| 2) Basis LS-60HR (manufactured by The Nisshin Oil Mills, Ltd., composition: PC: 71%, PE: 11%, PI: N.D.) | | | | | | | | | | |

**Table 4**

| Comparative Examples | 13 | 14 | 15 | 16 | 17 | 18 | 19 | 20 | 21 | 22 | 23 | 24 | 25 |
|---|---|---|---|---|---|---|---|---|---|---|---|---|---|
| Syn. Ex. 1 | 1.6 | 1.6 | 1.2 | 1.8 | 1.8 | 1.8 | - | - | - | - | 2.4 | 1.6 | 1.3 |
| Syn. Ex. 5 | - | - | - | - | - | - | 0.6 | 0.7 | 0.1 | 0.3 | - | 0.4 | 0.6 |
| Syn. Comp. Ex. 5 | - | - | - | - | - | - | - | 1.1 | - | - | - | - | - |
| Syn. Comp. Ex. 3 | - | - | - | - | - | - | - | - | 0.8 | - | - | - | - |
| Syn. Comp. Ex. 4 | - | - | - | - | - | - | - | - | - | 1.5 | - | - | - |
| Syn. Comp. Ex. 6 | 0.3 | - | - | - | - | - | - | - | - | - | 0.3 | - | - |
| Syn. Comp. Ex. 10 | - | 0.3 | - | - | - | - | - | - | - | - | - - | - | - |
| Syn. Comp. Ex. 8 | - | - | 0.7 | - | - | - | - | - | - | - | - | - | - |
| Syn. Comp. Ex. 9 | - | - | - | 0.1 | - | - | - | - | - | - | - | - | - |
| Glyceryl monooleate | - | - | - | - | 0.1 | - | - | - | - | - | - | - | - |
| EO (20 mol) sorbitan monooleate | - | - | - | - | - | - | 1.2 | - | - | - | - | - | - |
| Hydrogenated soybean phospholipid | 0.1 | 0.1 | 0.1 | 0.1 | 0.1 | 0.2 | 0.2 | 0.2 | 0.1 | 0.2 | 0.3 | - | 0.1 |
| Liquid paraffin | 2.0 | 2.0 | 2.0 | 2.0 | 2.0 | 2.0 | 2.0 | 2.0 | 2.0 | 2.0 | 0.5 | 2.0 | 2.0 |
| Octyl palmitate | - | - | - | - | - | - | - | - | - | - | 0.5 | - | - |
| 1,3-butylene glycol | 10.0 | 10.0 | 10.0 | 10.0 | 10.0 | 10.0 | 10.0 | 10.0 | 10.0 | 10.0 | 10.0 | 10.0 | 10.0 |
| Purified water | 86.0 | 86.0 | 86.0 | 86.0 | 86.0 | 86.0 | 86.0 | 86.0 | 86.0 | 86.0 | 86.0 | 86.0 | 86.0 |
| Avg. particle diameter (nm) | 52.0 | 51.0 | 63.3 | 57.5 | 48.0 | 123 | Precip. | 86.0 | 353 | 51.0 | 32.0 | 56.0 | 115 |
| Change in appearance | ○ | Δ | ○ | ○ | ○ | | - | | | ○ | ○ | ○ | Δ |
| Transmittance after production (%T) | 85.0 | 84.0 | 78.5 | 83.5 | 90.6 | 38.5 | - | 48.8 | 0 | 83.4 | 88.6 | 82.4 | 22.0 |
| Transmittance after 1 month (%T) | 78.5 | 65.3 | 73.5 | 79.8 | 65.3 | 0 | - | 0 | 0 | 79.5 | 84.4 | 76.3 | 0 |
| Transmittance stability | Δ | | Δ | Δ | | | - | | | Δ | Δ | Δ | |
| pH after production | 6.12 | 6.20 | 6.32 | 6.28 | 6.02 | 6.10 | - | 6.22 | 6.11 | 6.33 | 6.14 | 6.15 | 6.34 |

| | | | | | | | | | | | | | |
|---|---|---|---|---|---|---|---|---|---|---|---|---|---|
| pH after 1 month | 5.74 | 5.63 | 5.98 | 5.90 | 5.46 | 5.23 | - | 5.44 | 4.85 | 5.99 | 5.78 | 5.77 | 5.52 |
| pH stability | Δ | Δ | Δ | Δ | | | - | | | Δ | Δ | Δ | |
| ∗ Units: g | | | | | | | | | | | | | |

As is clear from Table 3, the lotions of Examples 9 through 18 exhibited superior emulsification stability, transmittance stability and pH stability. In contrast, the lotions of Comparative Examples 13 through 25 were inferior to the lotions of Examples 9 through 18 for all parameters. As a result, the effect of the present invention was confirmed.

### Example 19 - Emollient Lotion

An emollient lotion was prepared according to the blend indicated below. Namely, ingredients (1) through (6) were mixed and dissolved with a stirrer at 65°C followed by pouring in ingredients (7) through (9) at 65°C, and then cooling to 30°C and removing the emulsion to obtain a lotion. The lotion was evaluated in compliance with Examples 9 through 18, and the average particle diameter was 46.3 nm, the appearance was ○, transmittance stability was ⓞ (immediately after: 83.6%T, after 1 month: 83.2%T) and pH stability was ⓞ (immediately after 6.34, after 1 month: 6.25).

| | | |
|---|---|---|
| (1) | Synthesis Example 3 | 2.0 |
| (2) | Synthesis Example 8 | 0.4 |
| (3) | Hydrogenated soybean phospholipid²⁾ | 0.6 |
| (4) | Squalane | 2.0 |
| (5) | Cetyl octanoate | 0.8 |
| (6) | Methylparaben | 0.1 |
| (7) | Propylene glycol | 16.0 |
| (8) | Glycerin | 4.0 |
| (9) | Purified water | 64.1 |
| (10) | 1% aqueous sodium hyaluronate solution | 10.0 |
| | Total | 100.0 g |

### Example 20 - Emollient Lotion

A lotion was obtained in the same manner as Example 11 with the exception of replacing ingredient (4) in the blend of Example 19 with 1,3-butylene glycol, and replacing ingredient (8) with 3-methyl-1,3-butylene glycol. The lotion was evaluated in compliance with Examples 9 through 18, and the average particle diameter was 48.3 nm, the appearance was ○, transmittance stability was ○ (immediately after: 85.3%T, after 1 month: 83.4%T) and pH stability was ⓞ (immediately after 6.23, after 1 month: 6.17).

### Comparative Example 26

When a lotion was obtained in the same manner as Example 20 with the exception of replacing ingredient (3) in the blend of Example 20 with glyceryl monooleate, the average particle diameter was 52.3 nm, the appearance was ○, transmittance stability was (immediately after: 82.0%T, after 1 month: 0%T) and pH stability was (immediately after 6.20, after 1 month: 5.03).

### Example 21 - Emollient Lotion

A lotion was obtained in the same manner as Example 19 with the exception of replacing ingredient (5) in the blend of Example 19 with phytosteryl oleate. The lotion was evaluated in compliance with Examples 9 through 18, and the average particle diameter was 40.3 nm, the appearance was ○, transmittance stability was ⓞ (immediately after: 91.3%T, after 1 month: 91.1 %T) and pH stability was ⓞ (immediately after 6.26, after 1 month: 6.25).

### Example 22 - Medicinal Essence

A medicinal beauty lotion was prepared according to the blend indicated below. Namely, ingredients (1) through (9) were mixed and dissolved with a stirrer at 65°C followed by pouring in ingredient (10) at 65°C, followed by adding ingredients (11) and (12), cooling to 30°C and removing the emulsion to obtain a beauty lotion. The beauty lotion was evaluated in compliance with Examples 9 through 18, and the average particle diameter was 74.3 nm, the appearance was ○, transmittance stability was ⓞ (immediately after: 81.5%T, after 1 month: 80.7%T) and pH stability was ⓞ (immediately after 6.13, after 1 month: 6.00).

| | | |
|---|---|---|
| (1) | Synthesis Example 3 | 0.4 |
| (2) | Synthesis Example 4 | 0.2 |
| (3) | Synthesis Example 7 | 0.2 |
| (4) | High-purity hydrogenated soybean phospholipid³⁾ | 1.2 |
| (5) | Lyzosoybean phospholipid⁴⁾ | 0.2 |
| (6) | Tocopherol acetate | 0.1 |
| (7) | Propylene glycol | 12.0 |
| (8) | Glycerin | 4.0 |
| (9) | Methylparaben | 0.1 |
| (10) | Purified water | 61.5 |
| (11) | 1% aqueous xanthan gum solution⁵⁾ | 10.0 |
| (12) | Quince extract | 10.0 |
| | Total | 100.0 g |

| | | |
|---|---|---|
| 3) Basis LS-100H (manufactured by The Nisshin Oil Mills, Ltd., composition: PC: 97%, PE: N.D., PI: N.D.) | | |
| 4) Basis LP-20E (manufactured by The Nisshin Oil Mills, Ltd., 80% hydrolyzed lyzolecithin) | | |
| 5) Nomucoat ZZ (manufactured by The Nisshin Oil Mills, Ltd., type that dissolves to transparency in water) | | |

### Example 23 - Emollient Lotion

A lotion was obtained in the same manner as Example 17 with the exception of using 1.8 g of component (1) in the blend of Example 17 and replacing component (2) in the blend of Example 17 with 0.6 g of Sunsoft Q-183Y (Taiyo Kagaku, Co., Ltd.) (polyglycerol stearic ester, HLB value: 10.2; component polyglycerol has an average degree of polymerization of 10, and is composed of 3% free glycerin, 5% dimer component, 7% trimer component, 8% tetramer component, 9% pentamer component, 10% hexamer component, 12% heptamer component, 21% octamer component, 12% nonamer component, 8% decamer component, and 5% undecamer and higher polymer component). The average particle diameter was 40.1 nm, the appearance was ○, transmittance stability was ⓞ (immediately after: 88.3%T, after 1 month: 88.1%T) and pH stability was ⓞ (immediately after 6.23, after 1 month: 6.17).

### INDUSTRIAL APPLICABILITY

The present invention is a cosmetic emulsion preparation and agent for external use having for its emulsification base a polyglycerol fatty acid ester, and can be used in the fields of cosmetics, quasi-drugs, drugs, household articles, and so forth. In particular, even if a polyglycerol fatty acid ester having a high HLB value is used so as to facilitate use of phase inversion emulsification in order to realize the characteristics in terms of physical properties of higher alcohols and monoalkyl glyceryl ethers, the present invention provides a cosmetic emulsion preparation and agent for external use that remain stable even when stored with the passage of time without exhibiting a remarkable decrease in hardness at high temperatures of 40°C or higher, do not eliminate water and remain stable without increasing in hardness with the passage of time at 0°C, and have satisfactory texture in appearance while vanishing smoothly without leaving behind any white residue on the skin when used.

In addition, according to the present invention, when blending with natural lecithin, which has the effect of enhancing emulsification stability, the problem of decreasing pH of cosmetics and agents for external use containing polyglycerol fatty acid ester and lecithin with the passage of time, which had been a problem in the prior art, was able to be solved.

Furthermore, with respect to the blend of the present invention, an emulsified composition is obtained even with another emulsification method such as dispersion emulsification, D phase inversion emulsification or liquid crystal emulsification other than phase inversion emulsification. Since similar effects are obtained with these methods as well particularly in cases in which hardness is not required, the application range of the present invention can be said to be extremely broad.

In addition, the present invention provides a transparent cosmetic emulsion preparation and agent for external use having for its emulsification base a polyglycerol fatty acid ester, and can be used in the fields of cosmetics, quasi-drugs, drugs, household articles, and so forth. This superior transparent cosmetic emulsion preparation and agent for external use exhibits emulsification stability in a transparent state without clouding or separation by oily ingredients, has pH stability without exhibiting any decreases in pH with the passage of time as is observed in aqueous compositions combining the use of polyglycerol fatty acid ester and lecithin, and is free of precipitation of fatty acid and precipitation of lecithin with the passage of time.

Furthermore, even if the transparent cosmetic emulsion preparation and agent for external use of the present invention has an extremely high concentration for the blended amount of oily ingredients in comparison with an ethylene oxide-based fine emulsion, such as a concentration of 3% to 10% by weight within the composition, they remain transparent and stable, and can also be applied to a transparent cosmetic emulsion preparation and agent for external use having a high level of emollient effects similar to moisturizers and creams that cannot be obtained with conventional beauty washes and lotions.

In addition, in terms of safety, the present invention can also be applied to products not containing antiseptic as an additive. In the past, in the case of containing one or a combination of two or more substances such as 1,3-butylene glycol, 3-methyl-1,3-butylene glycol, 1,2-pentanediol, propylene glycol, or 1,2-hexanediol, or in the case of preparing with an ethylene oxide-based surfactant, even if a transparent composition is obtained by a fine emulsion, when these are blended at a high concentration of, for example, 15% to 25% by weight, there was the problem of clouding with the passage of time. However, according to the present invention, a transparent cosmetic emulsion preparation and agent for external use having superior emulsification stability can be obtained even in the case of blending these substances.

## Claims

1. A cosmetic emulsion preparation and agent for external use comprising the following components (A), (B), and (C) as essential ingredients:
(A) a polyglycerol fatty acid ester as a hydrophilic surfactant in which an HLB value is 13 or more, an average degree of polymerization of a polyglycerol is 8 to 12, a total content of pentamer and hexamer is 50% by weight or more, a total content of tetramer and lower polymers is less than 20% by weight, and a fatty acid residue is a linear alkyl group having 14 to 22 carbon atoms;
(B) a polyglycerol fatty acid ester as lipophilic surfactant in which an HLB value is 5 to 11, an average degree of polymerization of a polyglycerol is 2 to 12, a total content of tetramer or lower polymer is 20% by weight or more, each of (2/3)n components being contained in an amount of at least 5% by weight or more, where n (n is 2 or more) is taken to be an average degree of polymerization (when (2/3)n is not a natural number, (2/3)n is considered to be a lowest natural number greater than (2/3)n), and a fatty acid residue is a linear alkyl group having 14 to 22 carbon atoms; and,
(C) a saturated linear alcohol having 16 to 22 carbon atoms and/or a linear monoalkyl glyceryl ether having 16 to 22 carbon atoms.

2. A transparent cosmetic emulsion preparation and agent for external use comprising the following components (D), (E), (F), and (G) as essential ingredients and an average particle diameter of the emulsion of 100 nm or less:
(D) a polyglycerol fatty acid ester as hydrophilic surfactant in which an HLB value is 13 or more, an average degree of polymerization of a polyglycerol is 8 to 12, a total content of pentamer and hexamer is 50% by weight or more, a total content of tetramer and lower polymers is less than 20% by weight, and a fatty acid residue is a linear alkyl group having 14 to 22 carbon atoms;
(E) polyglycerol fatty acid ester as lipophilic surfactant in which an HLB value is 5 to 11, an average degree of polymerization of the polyglycerol is 2 to 12, a total content of tetramer or lower polymer is 20% by weight or more, each of (2/3)n components being contained in an amount of at least 5% by weight or more, where n (n is 2 or more) is taken to be an average degree of polymerization (when (2/3)n is not a natural number, (2/3)n is considered to be a lowest natural number greater than (2/3)n), and a fatty acid residue is a hydrocarbon group having 14 to 22 carbon atoms;
(F) lecithin; and,
(G) an oily ingredient.

3. A cosmetic emulsion preparation and agent for external use according to claim 1, wherein the polyglycerol in component (A) is a polyglycerol fatty acid ester containing a total content of pentamer and hexamer of 70% by weight or more.

4. A transparent cosmetic emulsion preparation and agent for external use according to claim 2, wherein the polyglycerol in component (D) is a polyglycerol fatty acid ester containing a total content of pentamer and hexamer of 70% by weight or more.

5. A cosmetic emulsion preparation and agent for external use according to claim 1, wherein the polyglycerol in component (A) is a polyglycerol fatty acid ester containing a total content of tetramer and lower polymers of 10% by weight or less.

6. A transparent cosmetic emulsion preparation and agent for external use according to claim 2, wherein the polyglycerol in component (D) is a polyglycerol fatty acid ester containing a total content of tetramer and lower polymers of 10% by weight or less.

7. A cosmetic emulsion preparation and agent for external use according to claim 1, wherein the polyglycerol in component (B) is a polyglycerol fatty acid ester with an average degree of polymerization of 6 to 12.

8. A transparent cosmetic emulsion preparation and agent for external use according to claim 2, wherein the polyglycerol in component (E) is a polyglycerol fatty acid ester with an average degree of polymerization of 6 to 12.

9. A cosmetic emulsion preparation and agent for external use according to claim 1, wherein the polyglycerol in component (B) is a polyglycerol fatty acid ester in which each of at least (1/2)n components is contained in an amount of 8% by weight or more, where n is taken to be an average degree of polymerization (when (1/2)n is not a natural number, a value is considered to be a lowest natural number greater than (1/2)n).

10. A transparent cosmetic emulsion preparation and agent for external use according to claim 2, wherein the polyglycerol in component (E) is a polyglycerol fatty acid ester in which each of at least (1/2)n components is contained in an amount of 8% by weight or more, where n is taken to be an average degree of polymerization (when (1/2)n is not a natural number, a value is considered to be a lowest natural number greater than (1/2)n.

11. A cosmetic emulsion preparation and agent for external use according to claim 1, wherein the polyglycerol in component (B) is a polyglycerol fatty acid ester in which each of at least (1/3)n components is contained in an amount of 10% by weight or more, where n is taken to be an average degree of polymerization (when (1/3)n is not a natural number, a value is considered to be a lowest natural number greater than (1/3)n).

12. A transparent cosmetic emulsion preparation and agent for external use according to claim 2, wherein the polyglycerol in component (E) is a polyglycerol fatty acid ester in which each of at least (1/3)n components is contained in an amount of 10% by weight or more, where n is taken to be an average degree of polymerization (when (1/3)n is not a natural number, a value is considered to be a lowest natural number greater than (1/3)n).

13. A cosmetic emulsion preparation and agent for external use according to claim 1, wherein each of (n-1) components is 3% by weight or more, where n is taken to be an average degree of polymerization of the polyglycerol in component (B).

14. A transparent cosmetic emulsion preparation and agent for external use according to claim 2, wherein each of (n-1) components is 3% by weight or more, when n is taken to be an average degree of polymerization of the polyglycerol in component (E).

15. A cosmetic emulsion preparation and agent for external use according to claim 1, wherein the polyglycerol in component (B) is a polyglycerol fatty acid ester in which a largest component contained therein is present in an amount 50% by weight or less.

16. A transparent cosmetic emulsion preparation and agent for external use according to claim 2, wherein the polyglycerol in component (E) is a polyglycerol fatty acid ester in which a largest component contained therein is present in an amount of 50% by weight or less.

17. A cosmetic emulsion preparation and agent for external use according to claim 1, wherein the polyglycerol in component (B) is a polyglycerol fatty acid ester in which a largest component contained therein is present in an amount of 30% by weight or less.

18. A transparent cosmetic emulsion preparation and agent for external use according to claim 2, wherein the polyglycerol in component (E) is a polyglycerol fatty acid ester in which a largest component contained therein is present in an amount of 30% by weight or less.

19. A cosmetic emulsion preparation and agent for external use according to claim 1, comprising lecithin.

20. A cosmetic emulsion preparation and agent for external use according to claim 1, comprising one type or two or more types of sterol ester.

21. A cosmetic emulsion preparation and agent for external use according to claim 1, comprising an oily ingredient.

22. A transparent cosmetic emulsion preparation and agent for external use according to claim 2, wherein component (F) has a phosphatidyl choline concentration of 50% by weight or more and a phosphatidyl inositol concentration of 3% by weight or less.

23. A transparent cosmetic emulsion preparation and agent for external use according to claim 2, comprises 15% to 25% by weight of one or a combination of two or more of 1,3-butylene glycol, 3-methyl-1,3-butylene glycol, 1,2-pentanediol, propylene glycol, or 1,2-hexanediol.

24. A transparent cosmetic emulsion preparation and agent for external use according to claim 2, wherein component (G) is one type of two or more types of higher fatty acid sterol ester in which the fatty acid residue has 14 to 22 carbon atoms.
